# EUROPEAN PATENT APPLICATION

(11) **EP 1 825 867 A1**
(43) Date of publication of application: **29.08.2007**
(21) Application number: 05814931.1
(22) Date of filing: 25.10.2005
(51) Int. Cl.: A61L 24/00

(54) **BICOMPONENT BIOADHESIVE FOR BIOMEDICAL USE**

(30) Priority: 26.10.2004 ES 200402573
(71) Applicant: Instituto Oftalmologico de Alicante, S.L., 03015 Alicante (ES)
(72) Inventor: ALIÓ SANZ, Jorge Luciano, E-03015 Alicante (ES); ABAD COLLADO, Marta, E-03015 Alicante (ES); SANCHEZ TORREGROSA, Alejandra, E-03015 Alicante (ES)
(74) Representative: Gislon, Gabriele
(86) International application number: PCT/ES2005/070150
(87) International publication number: WO 2006/048489

(57) **Abstract**

The present invention relates to new bicomponent bioadhesive formulations, with a synthetic part and an autologous biological part of blood origin composed of plasma rich in platelets and growth factors, and its use in biomedicine, preferably in ophthalmic surgery.

## Description

### TECHNICAL FIELD

The present invention relates to new bicomponent (made up of two components) bioadhesive formulations, one part synthetic and another part autologous biological (coming from the same body where it is going to be used) of blood origin consisting of plasma rich in platelets and growth factors. It is classified within the technical field intended to replace sutures in surgery.

### STATE OF THE ART

Throughout history, medicine has required materials to close traumatic and surgical wounds and for this reason the most harmless characteristics that have the least possible reaction and the most aesthetic scar have been searched for. The majority of sutures (reabsorbable or non-reabsorbable, synthetic, natural and metallic) have to a greater or lesser degree produced a reaction. This is due to the fact that any suture technique produces a micro-trauma and the tissues must respond in accordance with the re-epithelialisation and healing processes for repairing the wound. Generally, the traditional suture is made from synthetic or natural material, such as chromic nylon, silk or staples.

Numerous attempts are being made to replace sutures in the surgical field. The replacement of traditional sutures for bonding techniques using adhesives pursues multiple aims, since, on the one hand it tries to achieve a saving in surgical time for the surgeon, and on the other, it tries to avoid the additional trauma arising from the suture, and finally, it pursues a more uniform tissue bonding, where the tensile forces that tend to separate the edges of the wound are distributed homogeneously. This last point is particularly important in corneal eye surgery, since it could lead to the elimination of residual astigmatism and other refractive complications by enabling a more homogeneous distribution of the corneal healing process. The use of adhesion techniques in tissue bonding has been used in vascular surgery, ear, nose and throat, aesthetic surgery, plastic and reconstructive surgery, urology, gynaecology, neurology, orthopaedic and ophthalmic surgery, among others.

Significant improvements have been achieved in all fields in comparison with traditional sutures.

These improvements are summarised below:
- Once the adhesive bonding is formed there are no distortions in the wound.
- The tension is homogeneously distributed.
- The bonding process is rapid.
- The join is flexible and elastic.
- The adhesive allows access to internal areas of the wound, acting as a filling material.
- It decreases the risk of microbial contamination by completely sealing the wound or incision and in less time due to the application of platelets and growth factors which promote the closure of the wound or incision.
- Safety of the biological material, on being an autologous derivative, there will be no rejection risk, allergy or foreign body reaction.
- Once the adhesive bonding is made the excess adhesive becomes detached from the healed wound.
- The scar is more aesthetic.
- A saving in surgical time and health costs.
- Improved patient comfort on eliminating the need to remove the stitches.
Subsequent treatments are not required.

Currently there are two extensive ranges of adhesive for medical use: cyanoacrylate adhesives, of inorganic origin, and fibrin adhesives, of organic origin. Both have been used, although not routinely, for more than 15 years in the field of biomedicine.

Recently, Sharma et al [Ophthalmology 2003. Feb; 110(2): 291-8. "Fibrin Glue versus N-butyl 2-cyanoacrylate in corneal perforations*"]* compared the efficacy of butyl cyanoacrylate and fibrin adhesives, and found that both are effective for the closure of corneal perforations, and that fibrin adhesives also reduce corneal revascularisation. They also promoted a faster recovery, but needed a considerable time to cure and plug the wound.

On the other hand, in patent ES2110370 *[Nuevas formulaciones adhesivas en base cianoacrílica, procedimiento para su preparación y aplicaciones (New cyanoacrylic based adhesive formulations, procedures for their preparation and application)]* an adhesive formulation based on cyanoacrylic is described in which alkyl cyanoacrylate is accompanied by an alkyl carboxyacrylate compound and provides sufficient strength for bonding the rectus muscle to the sclera in ophthalmology surgery of strabismus.

Patent ES2169677 *[Composición adhesiva de base acrílica, procedimiento para su preparación y aplicación en cirugía (A crylic based adhesive composition, a procedure for its preparation and application in surgery)]* describes an adhesive formulation for bonding biological tissues in surgery, consisting of an alkyl cyanoacrylate and 6-hydroxyhexyl acrylate that has a particular application in surgery of the conjunctiva.

Patent ES2194219 *[Procedimiento para la preparación de una cola de fibrina autóloga (Procedure for the preparation of an autologous fibrin glue)]* describes a procedure for the preparation of an autologous fibrin glue for surgical use, as well as a closure system prepared by using 3 different plastic bags connected to each other by means of sealed tubes.

As mentioned previously, adhesion techniques have been used, among others, for bonding sectioned tissues in superficial wounds, for bone prosthesis bonding, in renal and lung surgery in animals, in middle ear surgery, cardiovascular surgery, gynaecology, genitourinary, plastic, neurological and ear, nose and throat surgery.

Hewitt et Al [Ann Torca Surg 2001;71:1609-12. BioGlue Surgical Adhesive for Thoracic Aortic Repair During Coagulopathy: Efficacy and Histopathology*]* tested a new adhesive made from fibrin of bovine origin and glutaraldehyde (P010003) and observed that it notably reduced post-surgical bleeding (haemostatic effect) and that the inflammatory response was minimal.

Bishara et al [Angle Orthod 2001;71:466-469. Effect of Using a New Cyanoacrylate Adhesive on the Shear Bond Strength of Orthodontic Brackets*]* compared the efficacy of cyanoacrylate adhesives with conventional adhesive systems for fixing dental brackets, and observed that sealing was faster in the case of cyanoacrylate adhesives.

Mark et Al [Arch Ophtalmol 2003;121:1591-1595. A Light-Activated Surgical Adhesive Technique for Sutureless Ophthalmic Surgery*]* describe a new polymeric adhesive, that contains indocyanine green and is activated with laser light at 632 nm and also uses a bovine protein sealant. This adhesive has given good results in several ophthalmic surgery procedures.

Other bioadhesives for surgical purposes and capable of being activated by an external factor can be found in the following publications:
Nakayama Y, Matsuda T. Photocurable Surgical Tissue Adhesive Glues Composed of Photoreactive Gelatine and Polyethylene Glycol Diacrylate. J Biomed Mater Res (Appl Biomater) 48:511-521, 1999*.*
Walsh et Al. Laser-Cured Fibrinogen Glue to Repair Bleb Leaks in Rabbits. Arch Ophthalmol. 1998; 116; 199-202*.*
Kaufman et Al. Human Fibrin Tissue Adhesive for Sutureless Lamellar Keratoplasty and Scleral Patch Adhesion. A pilot study. Ophthalmology 2003; 110:2168-2172*.*

In the field of Ophthalmic Surgery, adhesives have been used to a very limited extent in surgery of the anterior and posterior segment for the following clinical applications:
- Sealing of traumatic perforations and corneal ulcers;
- Joining artificial membranes to the anterior and posterior surface of the ocular chamber (artificial epithelium and endothelium);
- Adhesives in prostheses that penetrate the cornea;
- Sealing of conjunctival perforations;
- Performing scleral loops without sutures.

Cyanoacrylate adhesives are monocomponent (consisting of one component), that rapidly polymerise in the presence of a weak base, such as the water of the tissues. The cyanoacrylate adhesives mostly used in medicine are butyl, isobutyl or octyl derivatives. These adhesives have been studied and tested in many surgical fields, and demonstrate an extraordinary adhesion, in a few seconds, to biological tissue. However, they do have several disadvantages that limit their use considerably:
- High reactivity against humid environments or substrates; polymerisation generates an exothermic reaction which produces high temperatures in local areas and in turn, cell and tissue necrosis.
- High rigidity of the polymer mesh, which causes ulceration of the surrounding tissue.
- The polymer produced is opaque and meshed in the form of needles.
- Increased toxicity and slow biodegradation. In a study carried out on experimental rabbits, 3-4 months after being instilled in the eyes of these rabbits, considerable quantities of adhesive still remained, which delayed the natural healing process.

One advantage of fibrin adhesives is that they lack this toxicity, have a rapid bioreabsorbability and sufficient biotolerance, although the adhesive bonding is not strong enough to keep the substrates of the two tissues bound where there are opposing tethering forces on these or the joins, or even shear strains.

Existing commercial fibrin adhesives also have a problem of possibly transmitting the human immunodeficiency virus (HIV), as well as other blood transmissible viral diseases, which has led to it being prohibited for use in some countries.

### DESCRIPTION OF THE INVENTION

In the preceding state-of-the art it has been demonstrated that despite all the advantages of adhesion techniques compared to sutures, the use of adhesive substances in the biomedical field is not a regularly practiced technique. This, in part, is due to the fact that, up until now, there has not been any adhesive described that combines the advantages of synthetic adhesives and biological adhesives, and minimises their disadvantages.

Synthetic adhesives provide structural and mechanical support, but they do not promote the regeneration of the tissue in the affected area. On the other hand, the currently used compounds of autologous biological origin can restore damaged tissue and improve its regeneration, but they still do not re-establish the physical strength of the tissue. The adhesive compositions object of the present invention resolve the above mentioned problems by the simultaneous presence of two effects: adhesive and regenerative.

In the present invention, two adhesive products are combined which provide a new bi-functional bioadhesive formulation, which by means of its synthetic part provides the necessary tethering force to keep the substrates bound, and by using the autologous biological part it strengthens the adhesion due to the intrinsic adhesive property of the platelets and improves the process of re-epithelialisation, healing and tissue regeneration. On being derived from autologous blood any problems of rejection, allergy or foreign body reaction can be ruled out.

The synthetic part of the adhesive is responsible for providing the initial "tack" or adhesiveness and also to provide the adhesive strength required to keep the substrates (biological-biological; biological-alloplastic) bound together and in this way, to close the wound or incision.

The biological part of this new bicomponent bioadhesive is plasma rich in platelets and autologous growth factors (from the selfsame patient), which, besides having bioadhesive properties due to its nature and composition, a fact that reinforces the bioadhesion of the substrates, its main function is to act as a bio-stimulator of tissue regeneration, thus accelerating the wound healing process.

Growth factors, sometimes called mitogens, as they induce the cell to grow and move on to the mitosis phase, are small polypeptides situated inside the platelets (α-granules), 50 to 100 amino acids in length and make up a family of proteins that function at low concentrations and have a short biological half life. They promote mitogenic activity in the mesenchymal cells of blood, bone, cartilage and connective tissue. When they are present in sufficient quantities, they stimulate the cell so that it enters into the growth and division cycle, promoting cell growth through potent effects on cell migration, differentiation and proliferation. Growth factors act by bonding to the receptors present on the surface of the membrane. Each growth factor will only be bound to the extracellular domain of its specific receptor and not to other growth factors.

Growth factors form part of one of three mechanisms that the cells possess to interrelate (complexes that join them together (e.g. desmosomes), low molecular weight molecules and proteins, being the main growth factors. The name growth factor is not strictly correct, as the function of these molecules is not only to promote cell growth, as they possess other important properties, as well as maintaining cell survival, they initiate mitogenesis, stimulate cell migration and produce changes in phenotypes that have an effect on cell invasion or apoptosis (programmed cell death).

All growth factors have receptors on the cells, which enable signals to be transmitted within them, these are tyrosine kinases or serine/threonine kinases.

Growth factors are grouped into several families:
- **PDGF:** platelet derived growth factor. Involved in cell repair, and in cell proliferation mechanisms. These factors aid angiogenesis (formation of blood vessels) by activating the microphages through the induction of the formation of new capillaries by the endothelial cells.
- **TGF-β(beta)**: transformed growth factor-beta. Helps in tissue formation by increasing the rate of cell proliferation. It improves extracellular matrix deposition, increasing synthesis and inhibiting degradation.
- **FGF**: fibroblast growth factors. They stimulate angiogenesis by a direct mechanism, by stimulating mitosis and migration of the endothelial cells in the tissue repair process.
- **VEGF**: vascular endothelial growth factor. This is a mitogen selective for endothelial cells and its importance is demonstrated by its angiogenic action.
- **IGF-I**: insulin growth factor. It is more abundant in bone tissue, it generates osteoblasts and stimulates the formation of bone, inducing cell proliferation, differentiation and collagen biosynthesis. It is also found in significant quantities in platelets, and when it is liberated, it is a potent chemotactic for vascular endothelial cells, causing an increase in neovascularisation of the wound. It is able to stimulate mitogenic activity and acts as a chemotactic factor.

The adhesive or bioadhesive bicomponent of the present invention has many advantages over the known adhesives for surgical use. On diluting or mixing the synthetic adhesive with the biological one a bioadhesive mixture is obtained that offers, among others, the following advantages:
- The adhesive capacity of the new bioadhesive can be optimised for the desired application by adjusting the relative quantities of the components and the most appropriate choice of the synthetic adhesive component.
- The generative and bio-stimulating capacity of the mixture can be adjusted to each particular case by increasing or decreasing the proportion of growth factors and platelets.
- Polymerisation of the mixture is rapid, but not immediate, allowing the "open time" of the adhesive to be adjusted to the desired application and the skill of the surgeon.
- Reticulated adhesives are transparent and are not rigid, therefore the tissues surrounding the adhesive joint will not suffer from any type of ulcerous injury.
- The biodegradation and bioreabsorbability of the polymerised adhesive occurs in a shorter time period since the amount of synthetic adhesive will be applied in lower amounts than those used when it is not combined with a biological adhesive and the biological adhesive will be rapidly absorbed by the animal or human body.
- The toxicity of the mixture is lower, since the biological origin part of the adhesive lacks toxicity since it is an autograft (the blood itself is a tissue, and comes from the same patient), and therefore the body is not going to recognise it as a foreign substance. At the same time the biological part will partially mask the synthetic substance against the immune system thus reducing the toxic effects of this.

The percentage of components of the bioadhesive mixture, as well as their dosing will be set by the specialist, depending on several factors: tissue type and characteristics, surface to bond, state of the wound, etc.

In a particular embodiment, the composition or bioadhesive bicomponent of the present invention may be applied topically in a quantity of 0.25 ml/cm² of tissue surface.

The synthetic part of the bioadhesive combination described in the present invention can be any adhesive substance or formulation capable of providing sufficient tethering strength to keep the substrates bound. Depending on the characteristics of the tissue substrates and the adhesive strength required for the biomedical application in question, one or other component/formulation will be chosen for the synthetic/biological adhesive mixture.

A partial list of synthetic adhesive components, but not limited to this, is shown below, they illustrate and give an example of the type of adhesives and formulations that could be employed in this mixture:

### 1) Bioadhesive mixture for moderate to highly hydrated tissues. Cyanoacrylate based adhesive compositions with low exothermicity (able to be activated by H₂O):

Alkyl cyanoacrylate monomers of the general formula (1): 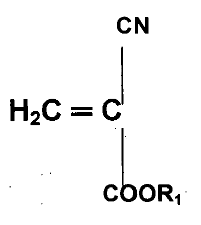 where R₁ is a C₁-C₂₅ alkyl group or a -(CH₂)ₙ-O-R₂ group, where R₂ is a C₁-C₂₅ alkyl group and n is a whole number between 1 and 25, both included.

The cyanoacrylic monomers are diluted in organic plasticising substances such as C₁-C₂₅ alkyl cyanoacetates, although substances with an ester type structure in general are also considered: C₁-C₂₅ alkyl or dialkyl maleates, C₁-C₂₅ alkyl or dialkyl adipates, etc., in volume proportions from 5% to 95% in the case of the cyanoacrylic monomer, so that they already constitute a novel series of highly flexible and low exothermal curing formulations in themselves, and in which the required adhesive properties can be adjusted (adhesive strength, flexibility, exothermicity and curing speed, specularity and hardness of the polymer formed, among others) by adjusting the quantities of alkyl cyanoacrylate and the plasticising substance, for the desired application.

The previous example forms part of the present invention as a new cyanoacrylic based adhesive formulation, of low curing exothermicity and high flexibility. These types of cyanoacrylic adhesive compositions cure by an anionic mechanism in the presence of a weak base, as in the case of water or an amine. For this reason these cyanoacrylic adhesive formulations are particularly appropriate for highly hydrated biological tissues where the water in the tissue itself, along with the ambient humidity, will be responsible for starting the polymerisation reaction. Unlike alkyl cyanoacrylate adhesives, which give rise to rigid and spiky polymers, these new adhesive formulations provide more flexible, transparent and softer polymers.

### 2) Bioadhesive mixture for poorly hydrated tissues. Thermo-activatable acrylic compositions (activated by applying a heat source):

Adhesive compositions based on one or several hydrophilic, water soluble, acrylic and/or methacrylic monomers, which have high polarity functional groups in their structure (hydroxyl, carbonyl, acetyl, halogen, amino, etc.) and are capable of solubilising small quantities of medium molecular weight polar polymer compounds or aqueous solutions of the same (E.g. polyvinylpyrrolidone, polyvinyl alcohol, etc.), so that the polymer constitutes a phase dispersed in a monomer/s matrix and which in the presence of an indicator, and a co-catalyst in required cases, are capable of curing by applying a precise and controlled heat source, at a temperature of not more than 60°C and adhering together with sufficient tethering strength, at least one of them being of biological origin and giving rise to the formation of an interwoven polymer material *in situ.*

This example forms part of the present invention and constitutes a synthetic adhesive which is activated by applying a heat source (thermoactivatable adhesive), which is also protected by this invention.

The monomers of the adhesive mixture are methacrylics or acrylics, with the following structure: 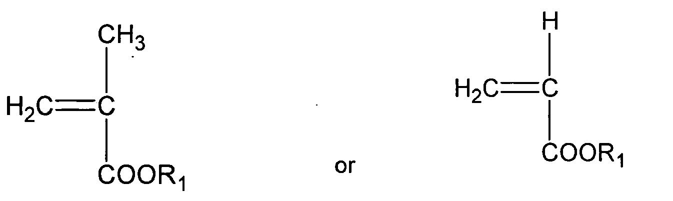 where R₁ is an alkyl or alkoxyl chain of 1 to 20 carbons with 1-19 functional groups whose atoms are electronegative and provide polarity to the molecule (OH, NH₂, Cl, SH₂, etc.).

The monomers are soluble or partially soluble in water and are combined with one another in different proportions (1%-99%) with the aim of adjusting the final properties of the cured adhesive in the biological tissue to the conditions required for each biomedical application: curing time, flexibility, exothermicity, transparency, etc.

The soluble monomer and/or mixture of monomers themselves are capable of dissolving small quantities of polymers of moderate polarity and moderate average molecular weight that are in powder or granulated form, or aqueous solutions of the same. Polyvinyl alcohol, polyvinylpyrrolidone, polymethylmethacrylate, etc., are examples of these polymers. The mixture of solid polymer and liquid monomer/s allows the viscosity of the adhesive mixture to be adjusted, and this will be between 10 - 2500 cPoises (MPa*s), so that the wetness of the surface to adhere is maximum and the application of the product is optimum.

The liquid adhesive composition will be thermoactivatable, it will form a solid product due to the action of the heat applied and for this it will need to include quantities of radical initiators and co-catalysts of not more than 1% in cases where required.

As examples of radical initiators that are used in these types of adhesive compositions, the following are mentioned: benzoyl peroxide/ N,N-dimethylaniline, ammonium persulphate/ sodium metabisulphite, potassium persulphate/ ascorbic acid, etc.

The present invention considers all those chemical substances that, in the presence of heat, are capable of forming radicals in a such a concentration that it does not inhibit polymerisation or curing of the monomers due to the presence of environmental oxygen or water from the tissues where the product may be applied, or by the inhibitors that join the monomers to guarantee their long term stability.

In difficult to adhere tissues, due to their particular position and structure, the adhesive compositions include other adhesion promoter additives (for example: siloxanes) in their formulation and also interweaving agents (acrylic monomers and/or multifunctional methacrylics) which will increase the strength of the adhesive bond at the same time as they decrease the curing time or open time of the adhesive.

The adhesive formulation is kept cold (<4°C) and in the presence of catalytic quantities of radical inhibitors (for example: 100 ppm hydroquinone, 100 ppm hydroquinone monoethyl ether, etc.) to guarantee its preservation.

The biological part of the bioadhesive combination described in the present invention can be any substance that is composed of platelets and/or growth factors, their concentration is not important, capable of supplying sufficient tethering strength to keep the substrates bound and induce tissue regeneration. Depending on the characteristics of the tissue substrates and the adhesive strength required for the biomedical application in question, one or other concentration of the biological adhesive will be chosen for the preparation of the synthetic/biological bioadhesive mixture.

### EXEMPLARY EMBODIMENTS OF THE INVENTION

The following specific examples provided here serve to further illustrate the adhesive components of this invention. These examples are included only for illustrative purposes and must not be interpreted as limitations to the invention that is claimed here. As will be understood by those skilled in the art, many variations and modifications can be made to the invention described in this document, which fall within the spirit and scope of this invention.

### Example 1: Acrylic based adhesive composition

### - Thermoactive adhesive composition 1.

The monomers and polymers (600 mg 2- Hydroxyethyl methacrylate, solution of 400 mg Polymethyl methacrylate in methyl methacrylate (1:16 w/w), 100 mg 3-glycidyloxypropyltrimethoxysilane) are mixed in a small polypropylene container, and mixed with a stirrer until the phases are completely miscible and the solid polymer is completely dissolved in the liquid monomers solution. This mixture can be kept in the cold for months, and a few minutes before its use the radical initiators and co-catalysts (20 mg Benzoyl peroxide, 30 mg N,N- dimethylaniline) are added, either in solid form by weight or in aqueous solution using a micropipette in the case of hydrophilic monomers which are soluble in water.

### - Thermoactive adhesive composition 2.

The monomers and the polymer (500 mg vinylpyrrolidone, 25% solution of 200 mg polyvinylpyrrolidone in phosphate buffer solution (PBS), 300 mg hydroxypropylacrylate, 100 mg ethylene glycidyl methacrylate) are mixed in a small polypropylene container and it is mixed with a stirrer until the phases are completely miscible and the solid polymer is completely dissolved in the liquid monomers solution. This mixture can be kept in the cold months, and a few minutes before its use, the radical initiators and co-catalysts are added, either in solid form by weight or in aqueous solution using a micropipette in the case of hydrophilic monomers which are soluble in water.

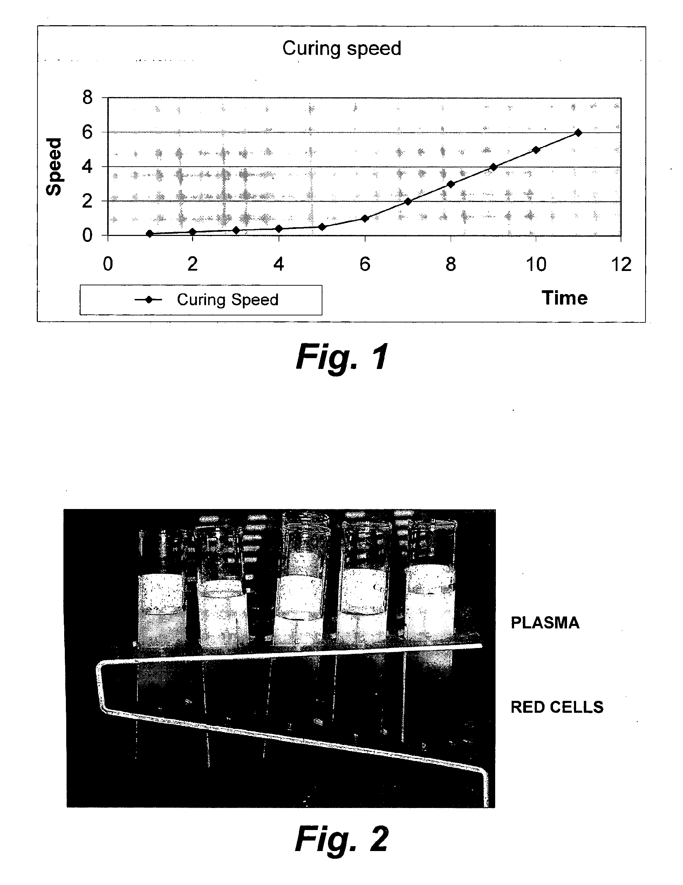

On adding the initiators, the monomer mixture slowly starts to reticulate, in such a way that it becomes more viscous. By applying the precise heat source which causes the slope of the polymerisation speed to increase with time and thus the adhesive bond can be activated and completely cured at the desired time, as can be seen in Figure 1, which shows a graph illustrating the polymerisation or curing speed against exposure time to a precise heat source, thus the adhesive bond can be activated and completely cured at the desired time.

### - Cyanoacrylic adhesive composition 1.

0.4 mL of butyl cyanoacrylate and 0.6 mL of butyl cyanoacetate are mixed in a small polypropylene container followed by 0.0020-0.0025% of a biocompatible colorant gentian violet (a mixture of crystal violet, C₂₅H₃₀CIN₃ MW 407.99 g/mol and methyl violet, C₂₄H₂₈CIN₃- MW 393.96 g/mol). This inorganic adhesive mixture is particularly suitable for well hydrated tissues and adhesion is instantaneous on the adhesive mixture coming into contact with the tissue fluids or remains of blood from the wound.

### Example 2: Platelet based adhesive composition

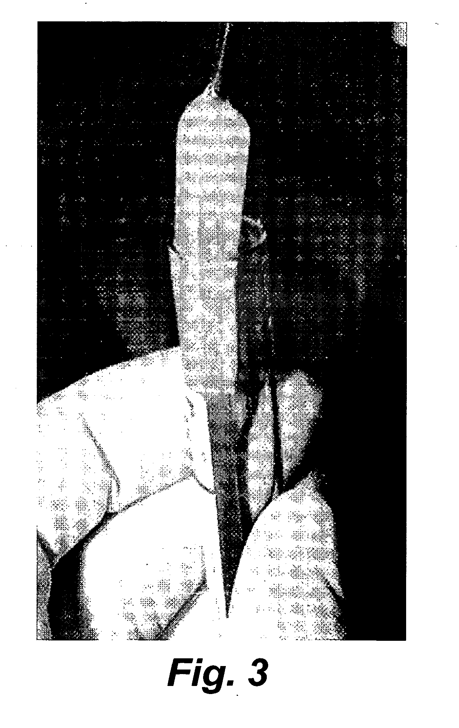

Autologous plasma with platelets and growth factors is obtained by venepuncture and centrifugation. A blood sample of 10-20 ml is taken, and placed in sterile tubes with 3.8% sodium citrate as anticoagulant. They are mixed gently by inverting the tubes 2 or 3 times. They are centrifuged, thus separating the plasma fraction and the red cells (see Figure 2). The platelets or concentrated platelets are activated using calcium chloride (sterile) and heat (37°C) to promote platelet aggregation, forming a clot which will be mixed with the synthetic bioadhesive (see Figure 3). A clot is formed within 3 to 5 minutes at room temperature. The speed of clot formation will vary depending on the quantity of platelets and the temperature. If it is brought to body temperature (37°C) clot formation is achieved in less time, between 1 and 2 minutes.

When the aforementioned platelets are activated, platelet activation begins and the content of these granules are liberated (the growth factors). The administration in the form of a clot provides adhesive support which confines the protein secretion *in situ* and promotes closure. Finally, this causes an increase in the rate of tissue generation and the healing process.

A partial list of the specific uses of the adhesive compositions covered by the invention is set out below. The different useful formulations are simply examples of the wide possible ranges of each component of the compositions that are the object of the invention:
According to a first aspect, the present invention relates to a bicomponent bioadhesive for biomedical applications which comprises of:
   a) a synthetic adhesive part;
   b) an autologous adhesive part.
According to a second aspect in the bicomponent bioadhesive the synthetic adhesive part can be any adhesive formulation with a capacity to bond two biological substrates to each other or a biological substrate and an inert substrate, such as a prosthesis, able to be activated by an external factor.
According to another important aspect in the bicomponent bioadhesive the synthetic adhesive part is selected from a group consisting of:
   - Cyanoacrylic based formulations diluted in plasticising compounds capable of being activated by humidity.
   - Acrylic formulations capable of being activated by heat.
   - Acrylic formulations capable of being activated by Ultraviolet Radiation.
   - Polyurethane adhesives capable of being activated by water.
   - Acrylic resins capable of being activated by blue light..
According to another important aspect in the bicomponent bioadhesive the biological adhesive part is selected from a group consisting of:
   - Autologous derivatives that include platelets.
   - Non-autologous preparations that include platelets.
   - Platelet concentrates.
   - Growth factors.
   - Growth factor concentrates.

According to an important aspect of the invention a Bioadhesive is referred to in which the biological adhesive part preferably consists of plasma rich in growth factors and platelets, in a range between 5% and 99% in volume with regard to the synthetic adhesive part.

According to an additional aspect of the invention the application of the bicomponent bioadhesive in surgery and medicine is referred to as a substitute for the traditional suture.

According to an important aspect of the invention the application of the bicomponent bioadhesive in orthopaedic reconstruction and repair is referred to as a substrate to adhere to bones, tendons, ligaments, meniscuses or muscles to each other, or any of those to alloplastic prosthetic materials. A useful formulation is 75% (v/v) ethyl cyanoacrylate/ethyl cyanoacetate 7:3 in volume, and 25% (v/v) of platelet and growth factors concentrate (1X, 2X, 4X, 6X, 8X, 10X, 20X,...units/µl (depending on the type of tissue) the normal blood platelet concentration of the patient).

According to an important aspect of the invention the application of the bicomponent bioadhesive in odontology is referred to as a substrate to adhere to a dental retainer structures, bridges, crowns or a filler to consolidate, repair and restore dental parts. A useful formulation is 85% (w/w) of thermoactive acrylic adhesive, 5% (w/w) siloxane and 10% (w/w) of plasma rich in super-concentrated platelets (e.g.: 20X or 40X).

According to an important aspect of the invention the application of the bicomponent bioadhesive in ophthalmology is referred to for curing perforations, lacerations or incisions, whether they have been induced surgically or associated with eye traumas or on the surface of the same. A useful formulation is 75% (v/v) of autologous P.R.G.F adhesive (Plasma Rich in Growth Factors) and 25% (v/v) octyl cyanoacrylate diluted in an octyl cyanoacetate plasticizer 1:1 (v/v).

According to an important aspect of the invention the application of the bicomponent bioadhesive in ophthalmology is referred to for re-attaching the retina to the back of the eye and underlying structure and/or by the direct modification of the vitreous humor or the space occupied by the vitreous humor that rests over the retina, and/or fixing alloplastic materials to the external surface of the eye (e.g., for deformities of the sclera) to modify the dimensions and the shape of the posterior chamber and influence the retina repair, or in the repair of retinal ruptures that result in traumatic or non-traumatic injuries. A useful formulation is 75% (v/v) of autologous P.R.G.F bioadhesive and 25% (v/v) ethyl cyanoacrylate diluted in ethyl cyanoacetate 7:3 (v/v).

According to an additional aspect of the invention the application of the bicomponent bioadhesive in ophthalmology is referred to for the repair or attaching of lenses (synthetic or natural) to adjacent tissues for implants, for the repair of the crystalline lens or the internal parts of the crystalline lens, and the fixing of other internal or external structures or substrates of the eye that require repair or reconstruction, such as alloplastic materials, tissues, episcleral musculature, contact lenses and similar. A useful formulation is autologous P.R.G.F bioadhesive 50% (v/v) and 50% (v/v) butyl cyanoacrylate diluted in butyl cyanoacetate plasticizer 1:1 (v/v).

According to an additional aspect of the invention the application of the bicomponent bioadhesive is referred to as an ophthalmic adhesive for the repair, construction, reconstruction an/or fixing of component part of the cornea (epithelium, endothelium, fibroblasts, collagen stroma) in keratoplasties. A useful formulation is 85% (w/w) of thermoactive acrylic adhesive, 5% (w/w) siloxane and 10% (w/w) plasma rich in super-concentrated platelets (e.g.: 20X or 40X).

According to an important aspect of the invention the application of the bicomponent bioadhesive is referred to as a medical adhesive for preparing an airtight adhesive formulation, to hermetically close wounds or incisions and adjacent areas where they have punctured by associated surgical closure devices and to reduce the leakage of fluid through them. A useful formulation is 75% (v/v) of P.R.G.F. autologous adhesive and 25% (v/v) ethyl cyanoacrylate diluted in ethyl cyanoacetate 7:3 (v/v).

According to an important aspect of the invention the application of the bicomponent bioadhesive is referred to as a medical adhesive for fixing tissue grafts or alloplastic grafts to soft tissues to repair wounds or as a prosthesis and to strengthen the suture of wounds in soft tissues, such as the liver, spleen, stomach, oesophagus, intestine, brain, skin, lungs, and anatomical structures similar to their subcomponents after an illness or traumatic or non-traumatic injury. The bioadhesive is also used in association with surgical closure methods, such as sutures, stitches and staples to help to hermetically close openings. A useful formulation is 85% (w/w) thermoactive acrylic adhesive, 5% (w/w) siloxane and 10% (w/w) plasma rich in super-concentrated platelets (e.g.: 20X or 40X).

According to an additional aspect of the invention the application of the bicomponent bioadhesive is referred to as a veterinary adhesive for repairing conjunctive tissues, bones and/or soft tissue wounds in animals. A useful formulation is 85% (w/w) thermoactive acrylic adhesive, 5% (w/w) siloxane and 10% (w/w) plasma rich in super-concentrated platelets (e.g.: 20X or 40X).

According to an important aspect of the invention the application of the bicomponent bioadhesive is referred to as a medical adhesive for implanting drugs, hormones, biological factors, medications or physiological and/or metabolic monitoring devices, antibiotics, cell isolates, cell sheets and similar, in intact tissues and/or in the sites of surgical and medical, therapeutic and repair processes, by means of fixing such agents directly to the adhesive polymer or the fixing of capsules that contain such agents.

## Claims

1. Bioadhesive for biomedical applications **characterised in that** it comprises:
a) a synthetic adhesive part; and
b) an autologous biological adhesive part from the selfsame patient.

2. Bioadhesive according to claim 1, **characterised in that** the synthetic adhesive part can be any formulation with a capacity to bond two biological substrates together or a biological substrate to an inert substrate capable of being activated by an external factor.

3. Bioadhesive according to any of claims 1 and 2, **characterised in that** the synthetic adhesive part is selected from a group comprised of:
- cyanoacrylic based formulations diluted in plasticising compounds capable of being activated by humidity;
- acrylic formulations capable of being activated by heat;
- acrylic formulations capable of being activated by ultraviolet radiation;
- polyurethane formulations capable of being activated by water;
- acrylic resins capable of being activated by blue light.

4. Bioadhesive according to claim 1, **characterised in that** the autologous biological adhesive part is selected from a group comprised of:
- autologous derivatives that include platelets;
- non-autologous preparations that include platelets.
- platelet concentrates;
- growth factors;
- growth factor concentrates.

5. Bioadhesive according to any of claims 1 to 4, **characterised in that** the autologous biological adhesive part preferably includes plasma rich in growth factors and platelets, in a range between 5% and 99% in volume with regard to the synthetic adhesive part.

6. Preparation of a bioadhesive according to any of claims 1 to 4 for its application in surgery and medicine as a substitute for the traditional suture.

7. Application of the bioadhesive according to any of claims 1 to 4 for the manufacture of an adhesive for orthopaedic repair and reconstruction as a substrate to bond bones, tendons, ligaments, meniscuses or muscles to each other or any of them to alloplastic materials.

8. Application of the bioadhesive according to any of claims 1 to 4 for the manufacture of an adhesive for odontology as a substrate to adhere to dental structure brackets, bridges, crowns or a filler to consolidate, repair and restore dental parts.

9. Application of the bioadhesive according to any of claims 1 to 4 for the manufacture of an adhesive to hermetically close wounds or incisions and the adjacent areas that have been punctured by associated surgical closure devices.

10. Application of the bioadhesive according to any of claims 1 to 4 for the manufacture of an adhesive for fixing tissue grafts or alloplastic grafts to soft tissues.

11. Application of the bioadhesive according to any of claims 1 to 4 for the manufacture of an adhesive to repair wounds and to promote the suture in soft tissues.

12. Application of the bioadhesive according to any of claims 1 to 4 for the manufacture of an adhesive for repairs to conjunctive tissues, bones and/or soft tissue wounds in animals.

13. Application of the bioadhesive according to any of claims 1 to 4 for the manufacture of an ophthalmic adhesive.

14. Application of the bioadhesive according to claim 12 for the manufacture of an ophthalmic adhesive for curing perforations, lacerations or incisions, whether they have been induced surgically or are related with trauma in the eye or on the surface of the same.

15. Application of the bioadhesive according to claim 12 for the manufacture of an ophthalmic adhesive for attaching the retina to the posterior part of the eye.

16. Application of the bioadhesive according to claim 12 for the manufacture of an ophthalmic adhesive for the modification of the dimensions and shape of the posterior chamber of the eye.

17. Application of the bioadhesive according to claim 12 for the manufacture of an ophthalmic adhesive to help in the repair of the retina, or in the repair of retinal ruptures that result from traumatic or non-traumatic injuries.

18. Application of the bioadhesive according to claim 12 for the manufacture of an ophthalmic adhesive for the repair or fixing of synthetic or natural lenses to adjacent tissues for implants.

19. Application of the bioadhesive according to claim 12 for the manufacture of an ophthalmic adhesive for the repair of the crystalline capsule or the internal portions of the crystalline.

20. Application of the bioadhesive according to claim 12 for the manufacture of an ophthalmic adhesive for the fixing of other structures to internal or external substrates of the eye that require repair or reconstruction.

21. Application of the bioadhesive according to claim 12 for the manufacture of an ophthalmic adhesive for the repair, construction, reconstruction and/or fixing of component parts to the cornea in keratoplasties.

22. Application of the bioadhesive according to any of claims 1 to 4 for the manufacture of an adhesive to implant components selected from a group comprised of:
- drugs
- hormones
- biological factors
- physiological and/or metabolic monitoring devices
- antibiotics
- cell isolates
- cell sheets.
